(19) Europäisches Patentamt
European Patent Office
Office européen des brevets

(11) Veröffentlichungsnummer : **0 065 487**
**B1**

# (12) EUROPÄISCHE PATENTSCHRIFT

(45) Veröffentlichungstag der Patentschrift : **28.08.85**

(51) Int. Cl.⁴ : **C 07 C127/22**, A 01 N 47/34

(21) Anmeldenummer : **82810196.4**

(22) Anmeldetag : **10.05.82**

## (54) Phenylharnstoffderivat.

(30) Priorität : **14.05.81 CH 3144/81**

(43) Veröffentlichungstag der Anmeldung : **24.11.82 Patentblatt 82/47**

(45) Bekanntmachung des Hinweises auf die Patenterteilung : **28.08.85 Patentblatt 85/35**

(84) Benannte Vertragsstaaten : **AT BE CH DE FR IT LI NL**

(56) Entgegenhaltungen : **EP-A- 0 016 729**

(73) Patentinhaber : **CIBA-GEIGY AG**
**Postfach**
**CH-4002 Basel (CH)**

(72) Erfinder : **Ehrenfreund, Josef, Dr.**
**Langenhagweg 11**
**CH-4123 Allschwil (CH)**

EP 0 065 487 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die vorliegende Erfindung betrifft ein neues N-(p-Aminophenyl)-N'-benzoylharnstoffderivat, Verfahren zu seiner Herstellung und seine Verwendung in der Schädlingsbekämpfung.

Der erfindungsgemäss vorgeschlagene substituierte N-(p-Aminophenyl)-N'-benzoylharnstoff hat die Formel I

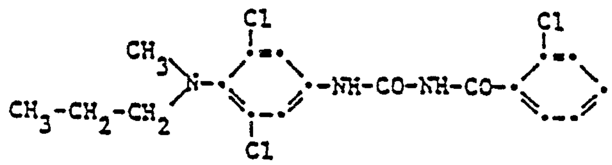

(I)

Gegenstand der Erfindung bilden auch die pflanzenphysiologisch und landwirtschaftlich geeigneten Salze der Verbindung der Formel I. Beispiele für zur Salzbildung geeignete Säuren sind Halogenwasserstoffsäuren, wie HCl, HBr, HJ, Phosphorsäure, Perchlorsäure, Rhodanwasserstoffsäure, Tetrafluorborsäure, Salpetersäure, Schwefelsäure, aliphatische und aromatische Sulfonsäure, Fettsäuren, Trichlor- und Trifluoressigsäure sowie mehrwertige organische Säuren, wie Oxalsäure, Malonsäure, Bernsteinsäure, Weinsäure, Adipinsäure und Zitronensäure.

Die Verbindung der Formel I kann nach an sich bekannten Verfahren hergestellt werden (vgl. u. a. die deutschen Offenlegungsschriften Nr. 2.123.236, 2.601.780).

So kann man z. B. die Verbindung der Formel I erhalten durch Umsetzung

a) der Verbindung der Formel II

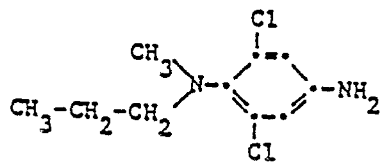

(II)

mit der Verbindung der Formel III

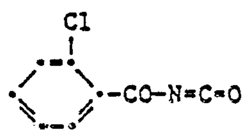

(III)

oder

b) der Verbindung der Formel IV

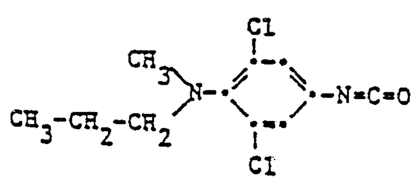

(IV)

mit der Verbindung der Formel V

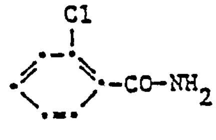

(V)

Die Herstellung der Salze der Verbindung der Formel I erfolgt nach allgemein bekannten Arbeitsweisen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z. B. Aether und ätherartige Verbindungen, wie Diäthyläther, Dipropyläther, Dibutyläther, Dioxan, Dimethoxyäthan und Tetrahydrofuran ; N,N-dialkylierte Carbonsäureamide ; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol ; Nitrile, wie Acetonitril oder Propionitril ; Dimethylsulfoxid sowie Ketone, z. B. Aceton, Methyläthylketon, Methylisopropylketon und Methylisobutylketon. Verfahren a) wird im allgemeinen bei einer Temperatur von − 10 bis 100 °C, vorzugsweise zwischen 15 und 25 °C, gegebenenfalls in Gegenwart einer organischen Base, z. B. Triäthylamin, durchgeführt. Die Durchführung von Verfahren b) erfolgt bei einer Temperatur von 0 bis 120 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Die Ausgangsstoffe der vorstehenden Formeln II, III, IV und V sind bekannt oder lassen sich, falls sie neu sind, analog bekannten Verfahren herstellen. So kann das p-Phenylendiamin der Formel II durch N-Alkylierung des 3,5-Dichlor-p-nitranilins und nachfolgende Reduktion oder katalytische Hydrierung der Nitrogruppe zur Aminogruppe hergestellt werden [vgl. z. B. Rec. *21*, 271 (1902) ; J. Am. Soc. *68*, 1604 (1946) ; J. Org. Chem. *11*, 378 (1946) ; Rec. *79*, 995 (1970)]. Das Isocyanat der Formel IV ist durch Phosgenisierung des N,N-substituierten p-Phenylendiamins der Formel II nach allgemein üblichen Arbeitsweisen erhältlich. Zum Benzoylisocyanat der Formel III kann man ausgehend von dem Benzonitril der Formel VI wie folgt gelangen (vgl. J. Agr. Food Chem. *21(3)*, 348-993) :

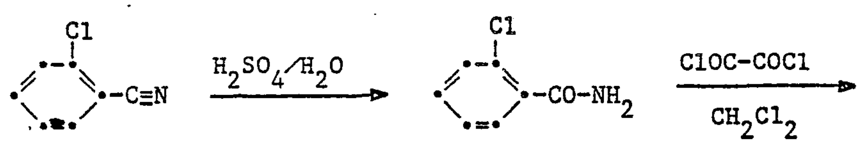

(III)

(VI) (V)

Es ist bereits bekannt, dass bestimmte N-Phenyl-N'-benzoylharnstoffe insektizide Eigenschaften besitzen (vgl. deutsche Offenlegungsschriften 2.123.236, 2.504.982, 2.537.413, 2.601.780 und 2.726.684, die belgischen Patentschriften 832.304, 843.906, 844.066 und 867.046 sowie die US-Patentschrift 4.089.975). Aus J. Agr. Food Chem. *21*, N° 3, 348 ff. (1973) sind weiterhin substituierte N-Phenyl-N'-2,6-dichlorbenzoylharnstoffe bekannt, die insektizide Eigenschaften aufweisen sollen. Auf Seite 353 dieser Veröffentlichung werden entsprechende N-(4-Dimethylamino)-phenyl- und N-(3-Chlor-4-dimethylamin)-phenylderivate erwähnt, die jedoch — wie aus der dort aufgeführter Tabelle III ersichtlich — nur unzureichende Insektizidwirkung zeigen.

Ferner werden in der veröffentlichten Europäischen Patentanmeldung Nr. 0 016 729 auf $N^1$-[3,5-Halogen-4-(N,N-dialkylamino)-phenyl]-$N^2$-halogenbenzoyl-harnstoffe beschrieben, wobei die erfindungsgemässe Verbindung der Formel I von dem dort angegebenen allgemeinen Umfang umfasst, aber als solche nicht offenbart wird. Es hat sich überraschenderweise herausgestellt, dass die vorliegende Verbindung der Formel I den aus der erwähnten Europäischen Patentanmeldung bekannten strukturähnlichen Verbindungen hinsichtlich ihrer insektiziden, insbesondere larviziden und oviziden, Wirkung überlegen ist (vgl. das nachfolgende Beispiel 12). Dies gilt auch für die in der Europäischen Patentanmeldung als besonders wirksam herausgestellten $N^1$-(p-Aminophenyl)-$N^2$-(2,6-difluorbenzoyl)-harnstoffe und $N^1$-(p-Allylaminophenyl)-$N^2$-(halogenbenzoyl)-harnstoffe. Es war nicht vorherzusehen, dass sich die erfindungsgemässe Verbindung der Formel I, deren Herstellung aus relativ leicht zugänglichen Ausgangsstoffen möglich ist, selbst bei geringen Aufwandmengen zur vollständigen Vertilgung von Populationen fressender Schadinsekten eignet, wobei auch die geringe Warmblütertoxizität und gute Pflanzenverträglichkeit der Verbindung zu erwähnen ist.

Insbesondere eignet sich die Verbindung der Formel I zur Bekämpfung von Insekten der Ordnungen : Lepidoptera, Coleoptera, Homoptera, Heteroptera, Diptera, Thysanoptera, Orthoptera, Anoplura, Siphonaptera, Mallophage, Thysanura, Isoptera, Psocoptera und Hymenoptera.

Neben ihrer günstigen Wirkung gegenüber Fliegen, wie z. B. Musca domestica, und Mückenlarven eignet sich die Verbindung der Formel I vor allem zur Bekämpfung von pflanzenschädigenden Frassinsekten, in Zier- und Nutzpflanzungen, insbesondere in Baumwollkulturen (z. B. gegen Spodoptera littoralis und Heliothis virescens) sowie in Obst- und Gemüsekulturen (z. B. gegen Laspeyresia pomonella, Leptinotarsa decemlineata und Pieris brassicae). Hervorzuheben ist besonders die larvizide, ovizide bzw. ovolarvizide Wirkung der Verbindung der Formel I. Wird die Verbindung der Formel I von adulten Insekten mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindung der Formel I eignet sich weiterhin zur Bekämpfung von ektoparasitären Insekten an Haus- und Nutztieren, z. B., durch Tier-, Stall- und Weidebehandlung.

Die gute insektizide Wirkung der erfindungsgemässen Verbindung der Formel I entspricht einer Abtötungsrate (Mortalität) von mindestens 50-60 % der erwähnten Schadinsekten.

Die Wirkung der erfindungsgemässen Verbindung bzw. der sie enthaltenden Mittel lässt sich durch

Zusatz von anderen Insektiziden und/oder Akariziden wesentlich verbreitern und an gegebene Umstände anpassen.

Als Zusätze kommen z. B. folgende Wirkstoffe in Betracht: organische Phosphorverbindungen, Nitrophenole und Derivate, Formamidine, Harnstoffe, Carbamate, chlorierte Kohlenwasserstoffe und Bacillus thuringienis-Präparate.

Es hat sich gezeigt, dass Präparate, die die erfindungsgemässe Verbindung der Formel I zusammen mit dem bekannten Insektizid « Galecron » [$N^2$-(4-Chlor-2-methyl-phenyl)-$N^1$,$N^1$-dimethyl-formamidin] enthalten, sich durch überraschend hohe insektizide Wirksamkeit und eine besonders breites Anwendungs-Spektrum auszeichnen.

Mit besonderem Vorteil kann man die Verbindung der Formel I auch mit Substanzen kombinieren, welche einen pestizid verstärkenden Effekt ausüben. Beispiele solcher Substanzen sind u. a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioat.

Die Verbindung der Formel I wird in unveränderter Form oder vorzugsweise zusammen mit den in der Formulierungstechnik üblichen Hilfsmitteln eingesetzt und wird daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d. h. die den Wirkstoff der Formel I, bzw. Kombinationen dieses Wirkstoffes mit andern Insektiziden oder Akariziden, und gegebenenfalls einen festen oder flüssigen Zusatzstoff enthaltenden Mitteln, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt bei Fraktionen $C_8$ bis $C_{12}$, wie z. B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxidiertes Kokosnussöl oder Sojaöl oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände verwendet werden.

Als oberflächenaktive Verbindungen kommen nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Tallöl gewonnen werden können. Ferner sind auch die Fettsäure-methyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z. B. das Na- oder Ca- Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z. B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und

6 bis 18 Kohlenstoffatome im Alkylrest der Alkylphenole enthalten können. Weiter geeignete nichtionische Tenside sind die wasserlöslichen 20 bis 250 Aethylenglykoläthergruppen und 10 bis 100 Propylenglykoläthergruppen enthaltend Polyläthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxyd-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyl-di-(2-chloräthyl)-äthylenammoniumbromid.

Die in der Formulierungstechnik gebräuchlichen Tenside sind u. a. in folgenden Publikationen beschrieben : « Mc Cutcheon's Detergents and Emulsifiers Annual » MC Publishing Corp., Ridgewood, New Jersey, 1979. Sisely and Wood, « Encyclopedia of Surface Active Agents », Chemical Publishing Co., Inc. New York.

Die pestiziden Zubereitungen enthalten in der Regel 0,1 bis 99 %, insbesondere 0,1 bis 95 %, Wirkstoff der Formel I oder Kombinationen dieses Wirkstoffes mit andern Insektiziden oder Akariziden, 1 bis 99,9 % eines festen oder flüssigen Zusatzstoffes und 0 bis 25 %, insbesondere, 0,1 bis 20 %, eines Tensides. Während als Handelsware eher konzentrierte Mittel bevorzugt werden, verwendet der Endverbraucher in der Regel Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für den Wirkstoff der Formel I resp. Kombinationen dieses Wirkstoffes mit andern Insektiziden oder Akariziden (% = Gewichtsprozent)

| 1. Spritzpulver | a) | b) | e) |
|---|---|---|---|
| Wirkstoff oder Wirkstoffkombination | 25 % | 50 % | 75 % |
| Na-Ligninsulfonat | 5 % | 5 % | — |
| Na-Laurylsulfat | 3 % | — | 5 % |
| Na-Diisobutylnaphthalinsulfonat | — | 6 % | 10 % |
| Octylphenolpolyäthylenglykoläther (7-8 Mol AeO) | — | 2 % | — |
| Hochdisperse Kieselsäure | 5 % | 10 % | 10 % |
| Kaolin | 62 % | 27 % | — |

Der Wirkstoff oder die Wirkstoffkombination werden mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | — |
| Kaolin | — | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Beispiel 1

5,0 g 3,5-Dichlor-(4-N-methyl-N-n-propylamino)-anilin werden in absolutem Aether gelöst und unter Kühlung und Ausschluss von Feuchtigkeit mit 3,9 g 2-Chlorbenzoylisocyanat versetzt. Der nach einiger Zeit ausgefallene Niederschlag wird abgesaugt und aus Aethanol umkristallisiert. Man erhält $N^1$-[3,5-Dichlor-(4-N-methyl-N-n-propylamino)-phenyl]-$N^2$-2-chlorbenzoylharnstoff vom Schmelzpunkt 134-136 °C.

## Beispiel 2 : Wirkung gegen Musca domestica

Je 509 frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Die erfindungsgemässe Verbindung gemäss Beispiel 1 zeigt gute Wirkung im obigen Test.

## Beispiel 3 : Wirkung gegen Lucilia sericata

Zu 9 ml eines Zuchtmediums wird bei 50 °C 1 ml einer 0,5 % Wirkstoff enthaltenden wässrigen Zubereitung gegeben. Nun werden ca. 30 frisch geschlüpfte Lucilia sericata-Larven zum Zuchtmedium gegeben und nach 48 und 96 Stunden wird die insektizide Wirkung durch Ermittlung der Abtötungsrate festgestellt.

Die erfindungsgemässe Verbindung gemäss Beispiel 1 zeigt in diesem Test gute Wirkung gegen Lucilia sericata.

Beispiel 4 : Wirkung gegen Aëdes aegypti

Auf die Oberfläche von 150 ml Wasser, das sich in einem Behälter befindet, wird so viel einer 0,1 %igen acetonischen Lösung des Wirkstoffes pipettiert, dass Konzentrationen von je 10, 5 und 1 ppm erhalten werden. Nach Verdunsten des Acetons wird der Behälter mit 30-40 3-tägigen Aëdes-Larven beschickt. Nach 1, 2 und 5 Tagen wird die Mortalität geprüft.

Die erfindungsgemässe Verbindung gemäss Beispiel 1 zeigt in diesem Test gute Wirkung gegen Aëdes aegypti.

Beispiel 5 : Wirkung gegen Heliothis virescens (Larven)

Ca. 25 cm hohe eingetopfte Baumwollpflanzen werden mit wässrigen Wirkstoffemulsionen besprüht, die den betreffenden Wirkstoff in Konzentrationen von 100, 50, 12,5, 3,0 und 0,75 ppm enthalten.

Nach dem Antrocknen des Sprühbelages werden die Baumwollpflanzen mit Heliothis virescens-Larven im dritten larvalen Stadium besiedelt. Der Versuch wird bei 24 °C und 60 % relativer Luftfeuchtigkeit durchgeführt. In Abständen von jeweils 24 Stunden wird die %-Mortalität der Test-Insekten bestimmt und der Frassschaden bonitiert (vgl. nachstehend Beispiel 12, Tabellen I und II).

Beispiel 6 : Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit :

a) 50 Larven von Spodoptera littoralis oder Heliothis virescens des ersten larvalen Stadiums ;

b) 20 Larven von Spodoptera littoralis oder Heliothis virescens des dritten larvalen Stadiums ;

c) zwei Eispiegeln von Spodoptera littoralis oder Heliothis virescens. Dazu wurden je 2 Blätter einer Pflanze in einem beidseitig mit Gaze verschlossen Plexiglaszylinder eingeschlossen ; zwei Eispiegel von Spodoptera oder ein Teil eines Baumwollblattes mit darauf abgelegten Eiern von Heliothis werden zu den eingeschlossenen Blättern gegeben.

Nach 4 bis 5 Tagen erfolgt die Auswertung gegenüber unbehandelten Kontrollen unter Berücksichtigung folgender Kriterien :

a) Anzahl der noch lebenden Larven,

b) Larvale Entwicklungs- und Häutungshemmung,

c) Frassschaden (Schabfrass und Lochfrass),

d) Schlupfrate (Anzahl der aus den Eiern geschlüpften Larven).

Die erfindungsgemässe Verbindung gemäss Beispiel 1 zeigt gute Gesamt-Wirksamkeit in obigem Test.

Beispiel 7: Ovizide Wirkung auf Spodoptera littoralis

Auf Filterpapier abgelegte Eier von Spodoptera littoralis wurden aus dem Papier ausgeschnitten und in eine 0,05 % Gew.-%ige Lösung des Wirkstoffes in einem Aceton-Wasser-Gemisch (1 : 1) getaucht. Die so behandelten Eiablagen werden dann aus diesem Gemisch herausgenommen und bei 21 °C und 60 % relativer Feuchtigkeit in Kunststoffschalen deponiert.

Nach 3 bis 4 Tagen wird die Schlupfrate, d. h. die Anzahl Larven, die sich aus den behandelten Eiern entwickelt hatten, bestimmt.

Die erfindungsgemässe Verbindung gemäss Beispiel 1 zeigt gute Wirkung im obigen Test.

Beispiel 8 : Ovizide Wirkung auf Epilachna varivestis

Es werden 20 Gew.-% Wirkstoff, 70 Gew.-% Xylol und 10 Gew.-% einer Mischung aus einem Reaktionsprodukt eines Alkylphenoles mit Aethylenoxid und Calcium-Dodecylbenzolsulfonat miteinander vermischt. Aus diesem Konzentrat werden wässrige Emulsionen enthaltend 800 und 1 600 ppm Wirkstoff hergestellt.

Jeweils ca. 100 auf Blätter von Phaseolus vulgaris frisch abgelegte Eier von Epilachna varivestis (mexikanischer Bohnenkäfer) werden mit den oben beschriebenen wässrigen Emulsionen (Konzentration 800 bzw. 1 600 ppm Wirkstoff) angefeuchtet und leicht getrocknet.

In einem gelüfteten Gefäss werden die behandelten Gelege solange gehalten, bis die gleichzeitig

angesetzten unbehandelten Kontrollen geschlüpft sind. Unter einem Binocular erfolgt Auswertung hinsichtlich der erzielten prozentualen Abtötung.

Die erfindungsgemässe Verbindung gemäss Beispiel 1 zeigt gute Wirkung in obigem Test.

Beispiel 9 : Ovizide Wirkung auf Heliothis virescens und Leptinotarsa decemlineata

Entsprechende Mengenanteile einer benetzbaren pulverförmigen Formulierung, enthaltend 25 Gew.-% des zu prüfenden Wirkstoffes, werden mit jeweils soviel Wasser vermischt, dass sich wässrige Emulsionen von ansteigender Wirkstoffkonzentration ergeben.

In diese wirkstoffhaltigen Emulsionen werden eintägige Eigelege von Heliothis auf Cellophan bzw. Eigelege von Leptinotarsa auf Kartoffelblättern während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt. Zur Auswertung wird die zur 100 %igen Abtötung der Eier erforderliche minimale Wirkstoffkonzentration bestimmt.

Die erfindungsgemässe Verbindung gemäss Beispiel 1 zeigt in diesem Test gute ovizide Wirkung gegen die geprüften Schädlinge.

Beispiel 10 : Ovizide Wirkung auf Laspeyresia pomonella

Abgelegte Eier von Laspeyresia pomonella, die nicht älter als 24 Stunden waren, werden auf Filterpapier für 1 Minute in acetonisch-wässrige Lösungen enthaltend 12,5, 50, 100 und 200 ppm des zu prüfenden Wirkstoffes eingetaucht. Nach dem Antrocknen der Lösung werden die Eier in Petrischalen ausgelegt und bei einer Temperatur von 28 °C belassen. Nach 6 Tagen wird mit Hilfe eines Binoculars der prozentuale Schlupf aus den behandelten Eiern, bzw. die erzielte %-Abtötung bewertet (vgl. nachstehend Beispiel 12, Tabelle I).

Beispiel 11 : Reproduktions- Beeinflussung von Anthonomus grandis

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel (wie z. B. « Actamer B 100 ») desinfiziert und dann in Schalen, die eine geeignete Larvaldiät enthielten, deponiert. Nach 7 Tagen wird untersucht, ob sich aus den deponierten Eiern Larven entwickelt haben.

Zur Ermittlung der Dauer eines die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und geschlüpfter Larven im Vergleich zu unbehandelten Kontrollen.

Die erfindungsgemässe Verbindung gemäss Beispiel 1 zeigt gute Wirkung in obigem Test.

Beispiel 12 : Biologische Ergebnisse

Die nachfolgenden Tabellen I und II zeigen Ergebnisse insektizider Vergleichsversuche auf der Basis der obigen biologischen Beispiele 5 und 10 unter Verwendung der erfindungsgemässen Verbindung und strukturell ähnlicher, aus der Europäischen Patentanmeldung Nr. 0 016 729 bekannten Verbindungen. Zur Bewertung der Versuche mit Larven von Heliothis virescens gemäss Beispiel 5 wurde die erzielte prozentuale Mortalität in Tabelle I und II angegeben. Die Auswertung der oviziden Versuche mit Laspeyresia pomonella gemäss Beispiel 10 erfolgte anhand der erhaltenen %-Mortalität unter Verwendung des folgenden Bewertungs-Index :

A. 80-100 % Mortalität bei einer Konzentration von 12,5 ppm der geprüften Verbindung
B. 80-100 % Mortalität bei einer Konzentration von 50 ppm der geprüften Verbindung
C. 80-100 % Mortalität bei einer Konzentration von 100 ppm der geprüften Verbindung
D. 80-100 % Mortalität bei einer Konzentration von 200 ppm der geprüften Verbindung.

Die in den Tabellen I und II aufgezeigten Ergebnisse lassen die Ueberlegenheit der erfindungsgemässen Verbindung gegenüber den Verbindungen gemäss der Europäischen Patentanmeldung Nr. 0 016 729 als Larvizid und Ovizid erkennen.

Tabelle I

| | geprüfte Verbindungen | %-Mortalität gegen Heliothis virescens (larvizid)-Beispiel 5 – Wirkstoffkonzentration 12,5 ppm | 3,0 ppm | 0,75 ppm | Wirkung gegen Laspeyresia pomonella (ovizid)-Beispiel 10 – Bewertungsindex |
|---|---|---|---|---|---|
| Verb. gemäss vorliegender Erfindung | 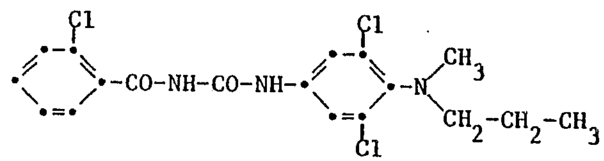 | 100 % | 100 % | 70 % | A |
| Verbindungen gemäss Europäischer Patentanmeldung Nr. 0 016 729 | 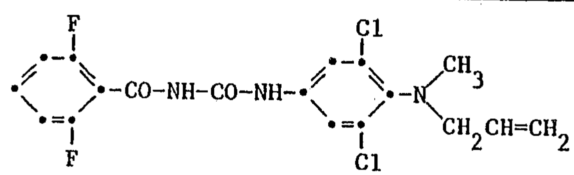 | 90 % | 50 % | 0 % | C |
| | 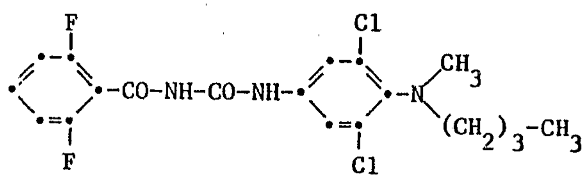 | 100 % | 100 % | 65 % | D |

Tabelle I (Fortsetzung)

| geprüfte Verbindungen | | %-Mortalität gegen Heliothis virescens (larvizid)-Beispiel 5 | | | Wirkung gegen Laspeyresia pomonella (ovizid)-Beispiel 10 |
|---|---|---|---|---|---|
| | | Wirkstoffkonzentration | | | |
| | | 12,5 ppm | 3,0 ppm | 0,75 ppm | Bewertungsindex |
| Verbindungen gemäss Europäischer Patentanmeldung Nr. 0 016 729 | 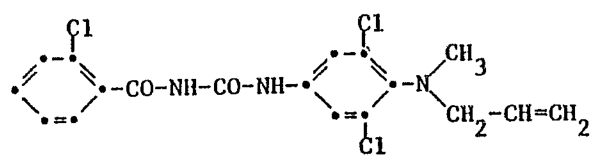 | 100 % | 92 % | 35 % | B |
| | 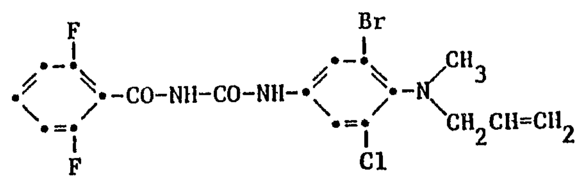 | 100 % | 97 % | 10 % | D |

Tabelle II

| | geprüfte Verbindungen | %-Mortalität gegen Heliothis virescens (larvizid)-Beispiel 5 Wirkstoffkonzentration 100 ppm | 50 ppm |
|---|---|---|---|
| Verb. gemäss vorliegender Erfindung | 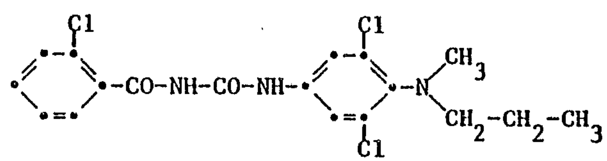 | 100 % | 100 % |
| Verbindungen gemäss Europäischer Patentanmeldung Nr. 0 016 729 | 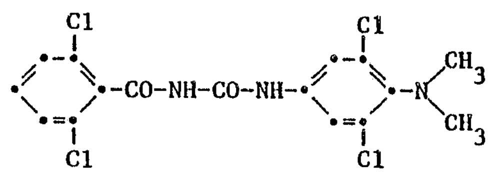 | 70 % | 30 % |
| | 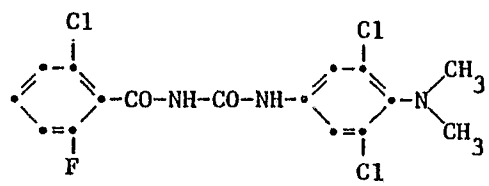 | 100 % | 80 % |

Tabelle II (Fortsetzung)

| geprüfte Verbindungen | | %-Mortalität gegen Heliothis virescens (larvizid)-Beispiel 5 Wirkstoffkonzentration 100 ppm | 50 ppm |
|---|---|---|---|
| Verbindungen gemäss Europäischer Patentanmeldung Nr. 0 016 729 | 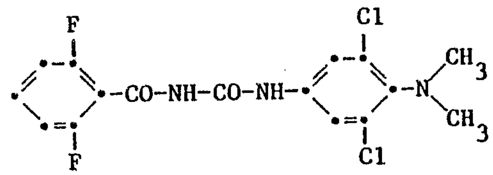 | 100% | 80% |
| | 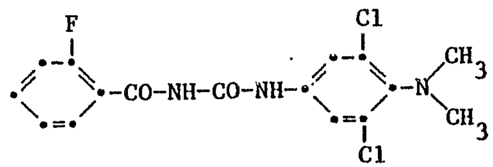 | 40% | 20% |
| | 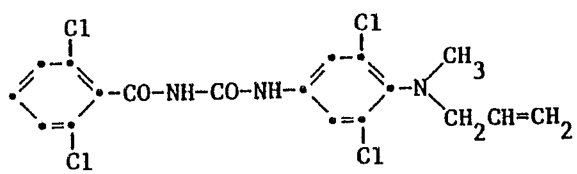 | 60% | 20% |

**Patentansprüche** (für die Vertragsstaaten : BE, CH, DE, FR, IT, LI, NL)

1. Verbindung der Formel I

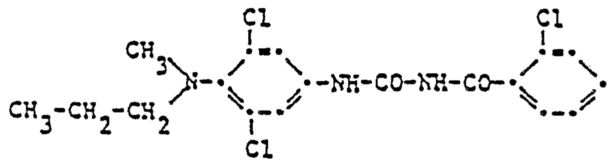

(I)

sowie deren Salze.

2. Verfahren zur Herstellung der Verbindung gemäss Anspruch 1, dadurch gekennzeichnet, dass man

a) die Verbindung der Formel II

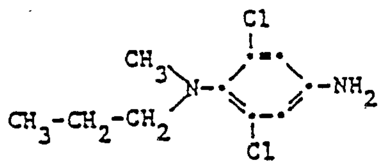

(II)

mit der Verbindung der Formel III

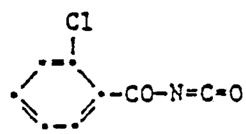

(III)

oder

b) die Verbindung der Formel IV

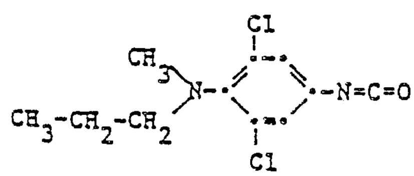

(IV)

mit der Verbindung der Formel V

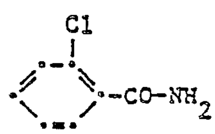

(V)

umsetzt und die erhaltene Verbindung der Formel I gegebenenfalls mit einer Säure in eine Salz überführt.

3. Schädlingsbekämpfungsmittel, welches als aktive Komponente die Verbindung gemäss Anspruch 1 oder ein Salz davon zusammen mit geeigneten Trägern und/oder anderen Zuschlagstoffen enthält.

4. Verwendung der Verbindung gemäss Anspruch 1 oder eines ihrer Salze zur Bekämpfung von Insekten.

5. Verwendung gemäss Anspruch 4 als Larvizid und/oder Ovizid zur Bekämpfung pflanzenschädigender Insekten.

6. Verwendung gemäss Anspruch 5 zur Bekämpfung pflanzenschädigender Frass-Insekten.

7. Verwendung gemäss Anspruch 6 zur Bekämpfung von Heliothis virescens und Lapeyresia pomonella.

**Patentansprüche** (für den Vertragsstaat AT)

1. Verfahren zur Herstellung der Verbindung der Formel I

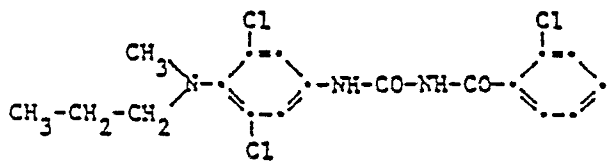

(I)

sowie deren Salze, dadurch gekennzeichnet, dass man

a) die Verbindung der Formel II

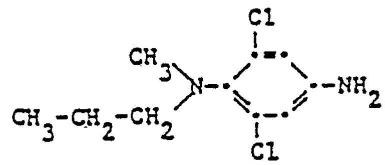

(II)

mit der Verbindung der Formel III

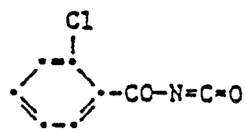

(III)

oder

b) die Verbindung der Formel IV

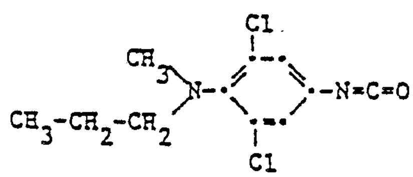

(IV)

mit der Verbindung der Formel V

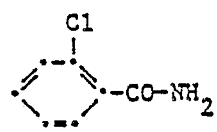

(V)

umsetzt und die erhaltene Verbindung der Formel I gegebenenfalls mit eine Säure in ein Salz überführt.

2. Schädlingsbekämpfungsmittel, welches als aktive Komponente die Verbindung der Formel I gemäss Anspruch 1 oder ein Salz davon zusammen mit geeigneten Trägern und/oder anderen Zuschlagstoffen enthält.

3. Verwendung der Verbindung der Formel I gemäss Anspruch 1 oder eines ihrer Salze zur Bekämpfung von Insekten.

4. Verwendung gemäss Anspruch 3 als Larvizid und/oder Ovizid zur Bekämpfung pflanzenschädigender Insekten.

5. Verwendung gemäss Anspruch 4 zur Bekämpfung pflanzenschädigender Frass-Insekten.

6. Verwendung gemäss Anspruch 5 zur Bekämpfung von Heliothis virescens und Lapeyresia pomonella.

**Claims** (for the Contracting States : BE, CH, DE, FR, IT, LI, NL)

1. The compound of the formula I

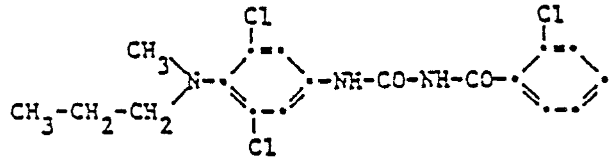

(I)

or a salt thereof.

2. A process for the preparation of the compound according to claim 1, which process comprises :

a) reacting the compound of the formula II

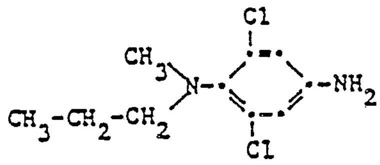

(II)

with the compound of the formula III

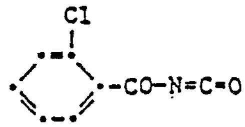

(III)

or

b) reacting the compound of the formula IV

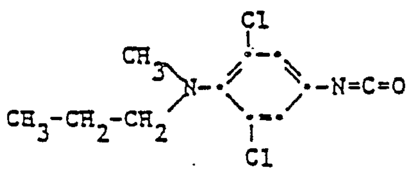

(IV)

with the compound of the formula V

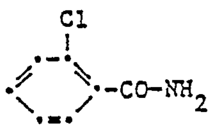

(V)

and optionally converting the resultant compound of the formula I with an acid into a salt.

3. A pesticidal composition which contains as active ingredient the compound according to claim 1 or a salt thereof, together with suitable carriers and/or other additives.

4. A method of controlling insects, which method comprises applying thereto or to the locus thereof a pesticidally effective amount of the compound according to claim 1 or of one of its salts.

5. A method according to claim 4, which method comprises the use of the compound according to claim 1 or of a salt thereof as a larvicide and/or ovicide for controlling insects which damage plants.

6. A method according to claim 5 of controlling insects which damage plants by eating.

7. A method according to claim 6 of controlling Heliothis virescens and Lapeyresia pomonella.

**Claims** (for the Contracting State AT)

1. A process for the preparation of the compound of the formula I

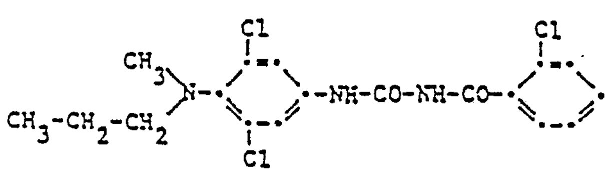

(I)

which process comprises

a) reacting the compound of the formula II

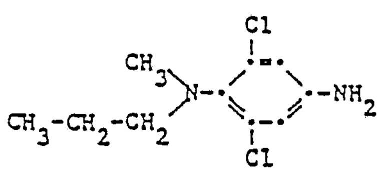

(II)

with the compound of the formula III

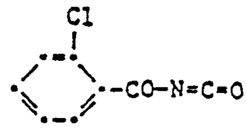

(III)

or

b) reacting the compound of the formula IV

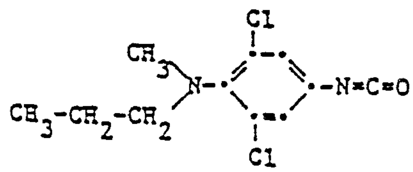

(IV)

with the compound of the formula V

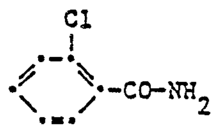

(V)

and optionally converting the resultant compound of the formula I with an acid into a salt.

2. A pesticidal composition which contains as active ingredient the compound of the formula I according to claim 1 or a salt thereof, together with suitable carriers and/or other additives.

3. A method of controlling insects, which method comprises applying thereto or to the locus thereof a pesticidally effective amount of the compound of the formula I according to claim 1 or of one of its salts.

4. A method according to claim 3, which method comprises the use of the compound of the formula I according to claim 1 or of a salt thereof as a larvicide and/or ovicide for controlling insects which damage plants.

5. A method according to claim 4 of controlling insects which damage plants by eating.

6. A method according to claim 5 of controlling Heliothis virescens and Lapeyresia pomonella.

**Revendications** (pour les Etats contractants : BE, CH, DE, FR, IT, LI, NL)

1. Composé de formule I

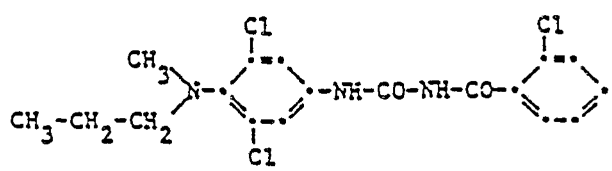

(I)

ainsi que ses sels.

2. Procédé de préparation du composé selon la revendication 1, caractérisé en ce qu'on fait réagir

a) le composé de formule II

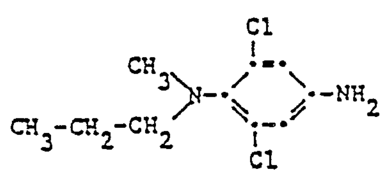

(II)

avec le composé de formule III

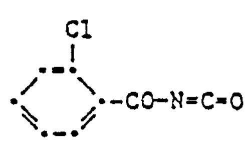

(III)

ou

b) le composé de formule IV

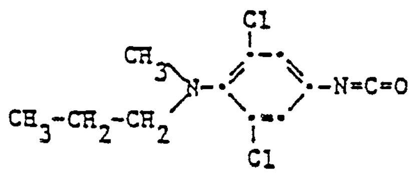

(IV)

avec le composé de formule V

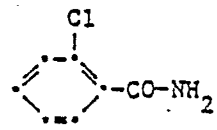

(V)

et en ce que le cas échéant on transforme le composé de formule I obtenu en un sel avec un acide.

3. Agent de lutte antiparasitaire contenant comme composant actif le composé selon la revendication 1 ou un de ses sels avec des supports et/ou autres additifs appropriés.

4. Application du composé selon la revendication 1 ou d'un de ses sels à la lutte contre les insectes.

5. Application selon la revendication 4 comme larvicide et/ou ovicide pour combattre les insectes qui attaquent les plantes.

6. Application selon la revendication 5 pour combattre les insectes phytophages.

7. Application selon la revendication 6 pour combattre Heliothis virescens et Lapeyresia pomonella.

**Revendications** (pour l'Etat contractant AT)

1. Procédé de préparation du composé de formule I

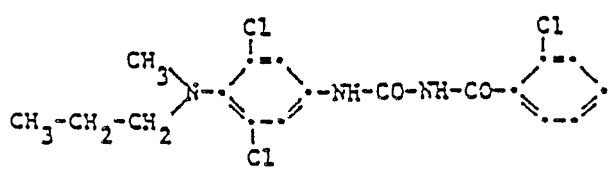

(I)

ainsi que de ses sels, caractérisé en ce qu'on fait réagir

a) le composé de formule II

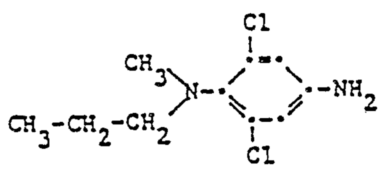

(II)

avec le composé de formule III

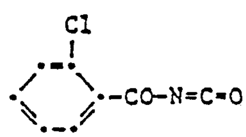

(III)

ou

b) le composé de formule IV

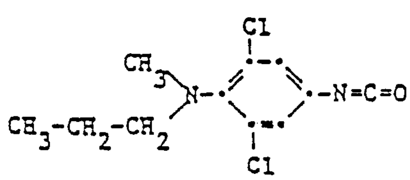

(IV)

avec le composé de formule V

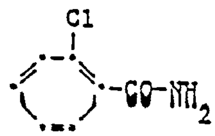

(V)

et en ce que le cas échéant on transforme le composé de formule I obtenu en un sel avec un acide.

2. Agent de lutte antiparasitaire contenant comme composant actif le composé de formule I selon la revendication 1 ou un de ses sels avec des supports et/ou autres additifs appropriés.

3. Application du composé de formule I selon la revendication 1 ou d'un de ses sels à la lutte contre les insectes.

4. Application selon la revendication 3 comme larvicide et/ou ovicide pour combattre les insectes qui attaquent les plantes.

5. Application selon la revendication 4 pour combattre les insectes phytophages.

6. Application selon la revendication 5 pour combattre Heliothis virescens et Lapeyresia pomonella.